# EUROPEAN PATENT SPECIFICATION

(11) **EP 1 451 206 B1**
(45) Date of publication and mention of the grant of the patent: **14.02.2007**
(21) Application number: 02792854.8
(22) Date of filing: 02.12.2002
(51) Int. Cl.: C07K 1/107, C07K 1/26, C07K 1/28

(54) **PROCESS FOR THE SELECTIVE ALKYLATION OF -SH GROUPS IN PROTEINS AND PEPTIDES FOR THE STUDY OF COMPLEX PROTEIN MIXTURES**
VERFAHREN ZUR SELEKTIVEN ALKYLIERUNG VON SH-GRUPPEN IN PROTEINEN UND PEPTIDEN ZUR UNTERSUCHUNG KOMPLEXER PROTEINMISCHUNGEN
PROCEDE D'ALKYLATION SELECTIVE DE GROUPES SH DANS DES PROTEINES ET DES PEPTIDES POUR L'ETUDE DE MELANGES DE PROTEINES COMPLEXES

(30) Priority: 05.12.2001 IT MI20012564
(43) Date of publication of application: 01.09.2004
(73) Proprietor: Bio-Rad Laboratories, Inc., Hercules, California 94547 (US)
(72) Inventor: Righetti, Pier Giorgio, 20129 Milano (IT)
(74) Representative: Jacob, Reuben Ellis
(86) International application number: PCT/EP2002/013593
(87) International publication number: WO 2003/048191

(56) References cited:
- WO-A-97/28185
- WO-A-99/49035
- US-A- 5 304 481
- YAN JX ET AL.: "Identification and quantitation of cysteine in proteins separated by gel electrophoresis" JOURNAL OF CHROMATOGRAPHY A, vol. 813, 1998, pages 187-200, XP004127079
- SHEWRY PR ET AL.: "Two-dimensional Separation of the Prolamins of Normal and High Lysine Barley (Hordeum vulgare L.)" JOURNAL OF EXPERIMENTAL BOTANY, vol. 28, no. 104, June 1977 (1977-06), pages 597-606, XP008019513
- HAMDAN M ET AL.: "Protein alkylation by acrylamide, its N-substituted derivatives and cross-linkers and its relevance to proteomics: A matrix assisted laser desorption/ionization-time of flight-mass spectrometry study" ELECTROPHORESIS, vol. 22, no. 9, May 2001 (2001-05), pages 1633-1644, XP008019511

## Description

### Description of the invention

The present invention refers to a process for the selective and efficient alkylation of -SH groups in proteins. The process of the invention is useful in a number of analytical techniques for protein analysis and characterization.

### Background of the invention

In the post-genomic era an emerging field is the science called "proteomics", i.e. a research area with the aim of the global analysis of gene expression, via a combination of methods for resolving, identifying, quantifying and characterizing all proteins present in a tissue, in a cellular lysate, in a biological fluid, in an entire organism (Wilkins, M.R., Williams, K.L., Appel, R.D., Hochstrasser, D.F., Eds., Proteome Research: New Frontiers in Functional Genomics, Springer, Berlin, 1997). The term "proteome" is a newly introduced word, which signifies precisely the entire set of proteins expressed by the genome. Since the proteome, even of a simple cell lysate, could be extremely complex (comprising several thousands of proteins), for its analysis one has adopted powerful separation methods called two-dimensional (2-D) maps, which couple a charge-based separation method (isoelectric focusing, IEF), in the first dimension, to a mass-based separation method (SDS-PAGE; polyacrylamide gel electrophoresis in presence of sodium dodecyl sulphate) in the second dimension. This method, introduced already in 1975 (O'Farrell, P.H., J. Biol. Chem. 250, 1975, 4007-4021), has been refined over the years with the advent of immobilized pH gradients (Righetti, P.G., *Immobilized pH Gradients: Theory and Methodology.* Elsevier, Amsterdam, 1990), of new surfactants (Chevallet, M. et al., T., *Electrophoresis* 19, 1998, 1901-1909), of new staining methods (Rabilloud, T., *Anal. Chem.* 72, 2000, 48A-55A).

The main problems in the analysis of complex proteins mixtures include: (a) the complete sample solubilization; (b) the elimination of artifacts during the various electrophoretic steps. In order to solve the first problem, new surfactants, of the amido-sulphobetaine type, in combination with chaotropic agents, such as urea/thiourea (Rabilloud, T. et al., J., *Electrophoresis* 18, 1997, 307-316), have been described. As for the elimination of artefacts, it has been recently demonstrated that the reduction and alkylation of disulfide bridges is a fundamental step for preventing the formation of spurious protein zones in the 2-D map. The sole reduction of disulfide bridges in naive proteins (typically obtained with an excess of 2-mercaptoethanol of dithiothreitol) cannot avoid artefacts, especially in the first IEF dimension. In fact, during the IEF separation, especially in an alkaline milieu, the reduced -SH groups spontaneously re-oxidize, generating artefactual bands, due to homo- and hetero-oligomers among the same or different polypeptide chains (Herbert, B. et al., *Electrophoresis* 22, 2001, 2046-2057). Thus, it has been proposed to reduce and alkylate proteins as a fundamental pre-treatment of the sample, prior to any separation step. The standard alkylating agent has always been iodoacetamide, recommended since the fifties by protein chemists and universally adopted in all protocols of analysis. Recent data, though, have demonstrated severe limitations of this alkylating agent:
(a) to start with, it is impossible to obtain 100% alkylation of all reduced -SH groups (Galvani, M.et al., *Electrophoresis* 22, 2001, 2058-2065), thus leaving intact a number of free -SH groups, potentially able to generate spurious bands. If the alkylation process is prolonged for long times (>6 bours), not only the reaction of free -SH groups does not progress, but non-selective alkylation of other reacting groups takes place, in particular of ε-amino groups of Lys;
(b) additionally, the alkylation reaction is strongly inhibited by the presence of numerous surfactants adopted for solubilizing complex protein mixtures;
(c) finally, it has been recently reported that iodoacetamide reacts rapidly with one of the solubilizing agents universally adopted until now in protein analysis, and precisely with thiourea. The addition of iodoacetamide to thiourea (present in a strong excess) is even more rapid than the addition to the -SH groups in proteins, a parasitic reaction which strongly quenches the reaction yield (Galvani, M. et al., *Electrophoresis* 22, 2001, 2066-2074).

### Description of the invention

It has now been found a process which overcomes all the above drawbacks.

The process of the invention is based on the finding that vinylpyridine reacts with the reduced -SH groups of proteins quantitatively and selectively (*i.e*. by avoiding side reaction with other protein groups, such as lysines, tyrosines, terminal -NH₂ groups). Moreover, vinylpyridine does not give spurious reactions with other components of the solubilizing mixture, such as thiourea and is not inhibited by the surfactants typically utilized in said solubilizing mixtures.

According to the invention there is provided a pre-treatment process for use in the pre-treatment of a proteome derived protein mixture for use in a separation technique comprising electrophoretic two-dimensional maps, said maps comprising a first dimension by isoelectric focusing (either conventional or in immobilized pH gradients) followed by a second SDS-PAGE dimension the process comprising the selective alkylation of -SH groups in a protein, by the reaction in neutral or alkaline milieu of said protein in a mixture comprising at least one chaotropic agent and at least one zwitterionic, neutral or anionic surfactant with a vinylpyridine,
wherein the percent alkylation of the total -SH groups is higher than 90 %.

### Description of the figures

### Figure 1

Alkylation kinetics of reduced bovine α-lactalbumin after incubation with 100 mM IAA at pH 9.0. The time points have been taken up to 24 hours. Panel a: control (m/z=14191); panel b: 2 min of incubation (the m/z 14653 represents the octa-alkylated peak); panel c: 2 hours of incubation; panel d: 24 hours of reaction time (the m/z 14707 peak represent the nona-alkylated species; the desired product, m/z=14652 is now the minor components among a highly heterogeneous series of products). Analysis by mass spectrometry in the MALDI-TOF mode.

### Figure 2

MALDI-TOF mass spectra of bovine α-lactalbumin after 1 h incubation with DMA (panel a) or 4-vinylpyridine (panel b), both in presence of the surfactant 2% Triton X-100. Note that, in panel b, the peak at m/z 15248 represents an adduct of LCA with the MALDI matrix, sinapinic acid.

### Figure 3

MALDI-TOF mass spectra of bovine α-lactalbumin after 1 h incubation with 2% amidosulphobetaine-14 and: (a) acrylamide, (b) 2-vinylpyridine and (c) 4-vinylpyridine. In all cases the reaction has been carried out at pH 7.0. Note, in both panels b and c, a single reaction channel of LCA with 2VP and 4VP, respectively. The higher order peaks (m/z 15248 and 15468. in b and c) represent adducts LCA with the MALDI matrix, sinapinic acid.

### Figure 4

Reaction of 4-vinylpyridinev(panels a and b) and of acrylamide (panel c) with the -SH groups of lysozyme. In (a) the reaction has been carried out at pH 9.0, whereas at pH 7.0 in panels (b) and (c).

### Figure 5

Isotopic ratio in the peptides of a1-acid glycoprotein (from rat sera) labelled with either light or heavy (hepta-deuterated) 2-vinylpyridine. After labelling, the two rat sera were mixed in a 70:30 (heavy/light) ratio and fractionated via two-dimensional maps. The zone of the α₁-acid glycoprotein (Mᵣ 21546, pI 5.0) was eluted, digested with trypsin and analysed by mass spectrometry in the MALDI-TOF mode. The insert shows two fragments at m/z 1235.0 and 1241.9. The difference in m/z of 6.9 coincides with the difference between the 2-d₇-VP and the non-deuterated 2-VP. This observation, together with the ratio of the relative intensities of the two signals (ca. 68:32) confirms that this peptide contains a single Cys residue which reflects the ratio of the two isotopic markers (70:30) in the original protein mixture.

### Detailed description of the invention

Examples of vinylpyridines for use in the process of the invention are 2- and 4-vinylpyridine, having the following formulas:
2-Vinylpyridine 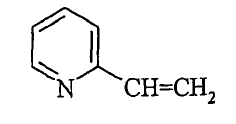
4-Vinylpyridine 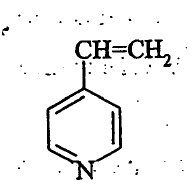 3-vinylpyridine may also be used, even though precaution should be used in view of its higher propensity to auto-polymerization.

The use of a vinylpyridine as alkylating agent for -SH groups in proteins has been generically mentioned by M.J. Dunn (in: *Gel Electrophoresis*: *Proteins*, Bio. Sci. Publ. Oxford, 1993). Alkylation of hordein fractions with 4-Vinylpyridine prior to 2-d Separation is disclosed by Shewry et al (Journal of Exp. Botany, Vol 28, No. 104 pp 597-606, June 1977), however there is no disclosure of such use in the presence of zwitterionic, neutral and anionic surfactants prior to 2d separation including isoelectric focusing.

The process according to the present invention allows for the alkylation of more than 90% of the -SH groups of a given protein, preferable more than 95% and even more preferably about 100%.

The process of the invention moreover can be utilized for protein alkylation both at alkaline and neutral pH value, it is compatible with the presence in the protein sample of surfactants, of zwitterionic (e.g., CHAPS, amidosulphobetaines), of neutral (Triton X-100, Nonidet P-40) of anionic (e.g., SDS) type. Vinylpyridines are additionally fully compatible with the chaotropic agents usually adopted for solubilizing complex protein mixtures, such as urea and thiourea, since they do not react with these compounds under the usual experimental conditions.

The protein, after reduction with a suitable reducing agent such as dithiotreitol, is reacted with vinylpyridine according to the invention at a temperature ranging from about 15 to about 30°C, usually, from about 20 to about 25°C, for a period of time from 30' to about 6 hours, usually from about 45' to 2 hours. The reaction solvent is not critical and it generally consists of an aqueous buffer such as a phosphate or a Tris-HCl buffer. An excess of the weakly basic compound will be generally used, for instance 100 mM for a 50 µM solution of the protein to be analyzed.

The vinylpyridines can optionally be deuterated, either totally (hepta-deuterated) or partially (in correspondence to the vinyl moiety or the pyridine ring), for the quantitative studies of protein synthesis in a biological sample, according to the method disclosed in Gygi, S.P., Aebersold, R., in *Proteomics: a Trend Guide,* Blackstock, W., Mann, M., eds., Elsevier, London, 2000, pp. 31-36).

The invention is illustrated in more detail in the following Examples, in comparison to iodoacetamide and to neutral derivatives of acrylamide, including N-substituted acrylamides.

### Comparative Example No. 1

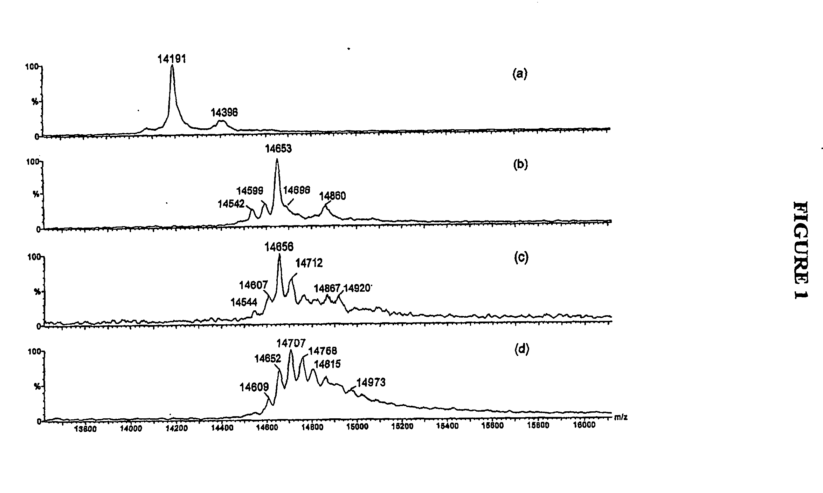

*Alkylation kinetics of bovine α-lactalbumin (LCA) with iodoacetamide (IAA).* A 50 µM solution of LCA in 50 µM Tris-acetate, pH 9.0 and 7 M urea, is reduced with 50 mM dithiothreitol (DTT) for 1 hour at 25°C. After reduction, the protein is incubated with 100 mM IAA and the alkylation kinetics followed up to 24 hours via mass spectrometry in the MALDI-TOF (matrix-assisted; laser desorption ionization - time of flight) mode. LCA contains, in the polypeptide chain, 8 cysteine residues, and therefore 8 -SH groups, which could potentially undergo alkylation. As shown in Fig. 1, already after 2 min the octa-alkylated derivatives represents only the 60% of all reaction products (panel b, peak at m/z 14653), the remaining 40% representing, hepta-, hexa- and penta-alkylated species, in this order. However, one can notice that, after 2 hours of reaction, the octa-alkylated derivative is barely increased to 70% (panel c, peak at m/z 14656), the remaining 30% being represented by derivatives of lower degree of alkylation. The reaction does not seem to progress any further for longer incubation times: after 24 hours, other groups present in the protein are alkylated instead (especially the ε-amino groups of lysine) (panel d; e.g., the peak at m/z 14707 is a nona-alkylated derivative, and represents the main peak; in addition one can notice peaks which could be deca-substituted and even at a higher degree of substitution; the final reaction product, in any event, is strongly heterogeneous and the desired product, at m/z 14652, represents now the minor component). The lack of quantitative reaction and the number of side reactions on other amino acid residues are strongly hampering a proper proteome analysis, since they make the correct identification of proteins difficult, presently mostly performed via mass spectrometric analysis. In addition, the extra reactions on lysines and other residues originate spurious spots in 2-D maps, in that they generate new protein isoforms having a different isoelectric point (pI).

### Example No. 2

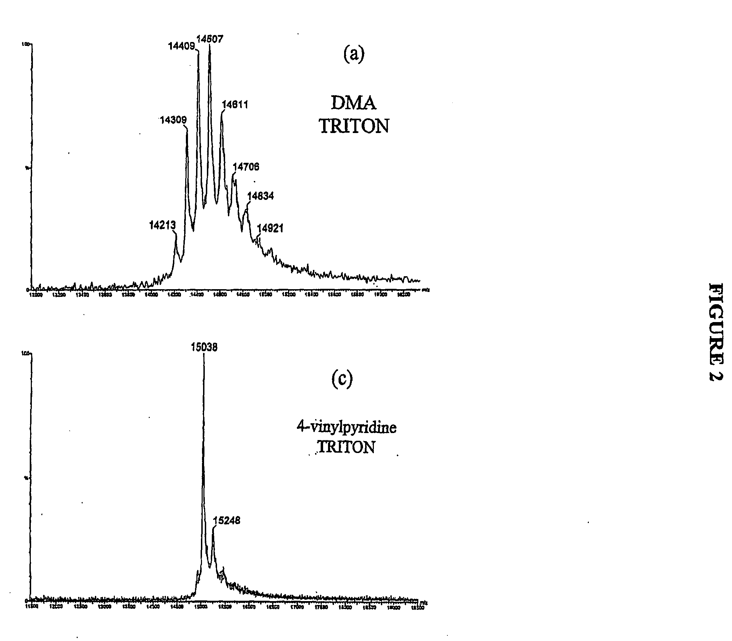

*Alkylation of bovine α-lactalbumin (LCA) with dimethyl acrylamide (DMA) and 4-vinylpyridine in presence of neutral surfactants.* A 50 µM solution of LCA in 50 mM Tris-acetate, pH 9.0 and 7 M urea, is reduced with 50 mM dithiothreitol (DTT) for 1 hour at 25°C. After reduction, the protein is incubated with 100 mM of either DMA or of 4-vinylpyridine (4VP) for 1 hour at 25°C. Both reactions are carried out in presence of 1% Triton X-100, one of the possible surfactants adopted in proteome analysis. The reaction products are monitored via mass spectrometry in the MALDI-TOF mode. As shown in Fig. 2, whereas in the case of 4VP only one reaction product is present (m/z 15083), corresponding to the LCA adduct with 8 4VP residues, in the case of DMA there are still some amounts of unreacted product (m/z 14213) accompanied by a whole series of reaction species, the most abundant components of which are tri- and tetra-alkylated species. 4VP turns out to be by far the most efficient reagent, able to carry out the reaction to full completion, while being unaffected by the inhibitory effect of surfactants.

### Example No. 3

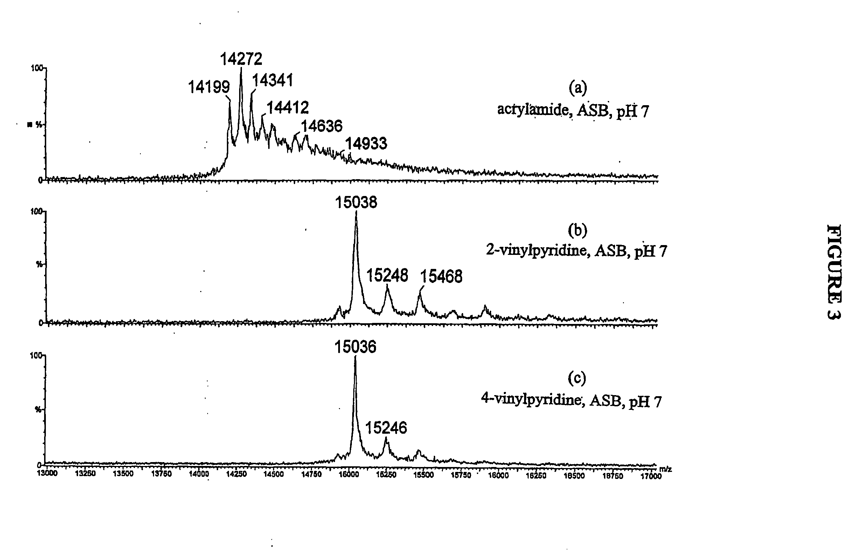

*Alkylation of bovine α-lactalbumin (LCA) with acrylamide and with 2-or 4-vinylpyridine in presence of zwitterionic surfactants at neutral pH.* Protein alkylation is typically carried out at pH values between 85 and 9.0, a pH which facilitates the reaction between the -S⁻ group (pK 8.3), negatively charged, and neutral agents such as IAA and acrylamide. At this pH value, though, and for long reaction times, spurious reaction take place, such as alkylation of the ε-amino groups of lysine. On the contrary, the 2- and 4-vinyl pyridines are weak bases (pKs *ca.* 5.2 to 5.6), which, at pH 5, exist in a protonated form, thus rendering them highly reactive with deprotonated -SH groups. Thus, an interesting reaction pH could be at pH around neutrality, i.e. half a way in between the pK values of the two reacting species. At pH *ca.* 7 both the alkylating agent and the -SH group would carry a partial positive and negative charge, respectively, which would render them highly reactive towards each other. A 50 µM solution of LCA in 50 mM phosphate buffer, pH 7.0 and 7 M urea, was reduced with 50 mM dithiothreitol (DTT) for 1 hour at 25°C. After reduction, the protein was incubated with 100 mM of either acrylamide or of 2- (2VP) or 4- (4VP) vinylpyridine for 1 hour at 25°C. Both reactions were carried out in presence of 1% amidosulphobetaine (ASB), a zwitterionic surfactants much in vogue today in proteome analysis. The reaction products were monitored via mass spectrometry in the MALDI-TOF mode. As shown in Fig. 3, whereas in the cases of 2VP and 4VP (panels b and c) only one reaction product is present at m/z 15038, corresponding to the adduct of LCA with 8 residues of 2VP or 4VP, in the case of acrylamide there still remains a substantial amount of unreacted product, accompanied by a series of reaction products, of which the most abundant species are tri- and tetra-alkylated compounds. This clearly shows how 2VP and 4VP represent by far the most efficient reagents, able to carry out the reaction to full completion, while being unaffected by the inhibitory effect of surfactants.

### Example No. 4

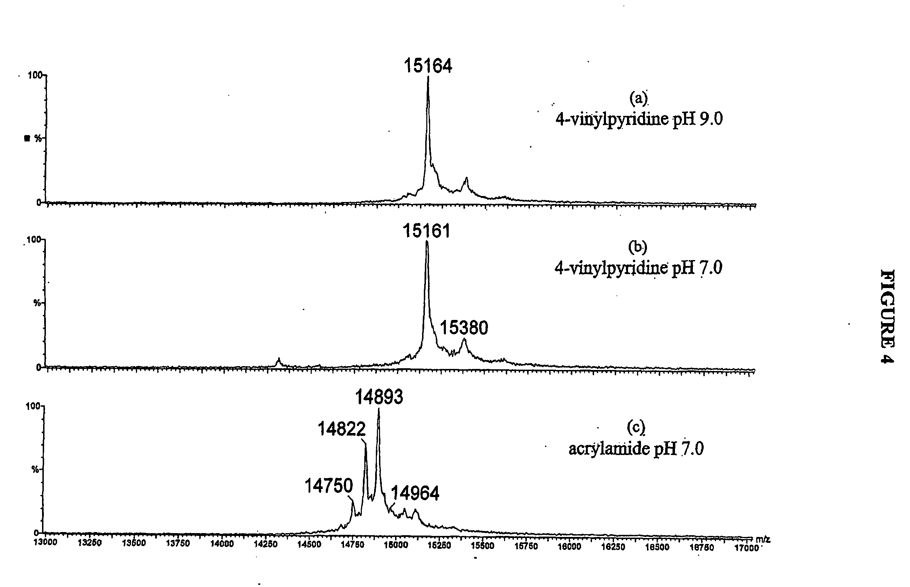

*Alkylation of lysozyme with acrylamide or 4-vinylpyridine at alkaline* and neutral pH values*.* We have also investigated whether the isoelectric point (pI) of the protein could influence the -SH group alkylation reaction. This could be a drawback, particularly in the case of 2VP and 4VP (due to their weakly basic characteristics), in that the excess of positive charges on such alkaline proteins could inhibit their access to the reaction sites. We have thus studied the behaviour of chicken egg lysozyme, a protein with a pI of *ca.* 10, also containing 8 cysteine residues in the polypeptide chain. A 50 µM solution of lysozyme in 50 mM phosphate buffer, pH 7.0, or in Tris-acetate buffer, pH 9.0, both in presence of 7 M urea, was reduced with 50 mM dithiothreitol (DTT) for 1 hour at 25°C. After reduction, the protein was incubated with 100 mM of either acrylamide or of 4VP for 1 hour at 25°C. The reaction products were monitored via mass spectrometry in the MALDI-TOF mode. As shown in Fig. 4, in the case of 4VP (panel a for the pH 9.0, panel b for the pH 7.0 reactions) only a single reaction product is present (m/z 15164), corresponding to the adduct of lysozyme with 8 residues of 4VP, whereas, in the case of acrylamide, the main peak is the m/z 14893 (corresponding to the adduct with 8 acrylamide residues), but this peak is contaminated by sizeable amounts of m/z 14750 and m/z 14822, corresponding to the hexa- and hepta-alkylated products, respectively. Here again, it is very clear how 4VP (and its analogue 2VP) are able to alkylate to 100% also basic proteins at unfavourable pH values, such as pH 7.0, a pH at which the positive charge of lysozyme would be considerably increased as compared to pH 9.0.

### Example No. 5

*Alkylation of proteins having large Mr values, with a high cysteine content, with IAA or 2 VP or 4 VP at neutral pH.* In the above examples, we have followed the alkylation of small-size proteins (13-15000 Da) and with an average content of -SH groups (8 Cys residues). It was of interest to follow the same reaction on medium to high mass proteins and with a higher content of Cys residues. Two interesting cases are represented by human serum albumin (HSA) (Mᵣ 66472, pI 5.7, containing 35 Cys residues) and by rabbit phosphorylase (Mᵣ 97158, pI 6.8, containing 36 Cys residues). Fifty µM solutions of HSA, or phosphorylase, in 50 mM phosphate buffer, pH 7.0 and 7 M urea, were reduced with 50 mM dithiothreitol (DTT) for 1 hour at 25°C. After reduction, the proteins were incubated with 100 mM of either acrylamide or of 2- (2VP) or 4- (4VP) vinylpyridine for 1 or 6 hours at 25°C. The reaction products were monitored via mass spectrometry in the MALDI-TOF mode. The results are reported in Table 1.

**Table 1. Alkylation with 2- o 4-VP or with IAA of proteins with medium to high Mr values**

| Protein | Mr | pI | Total Cys | IAA (1h)* | IA.A (6h)* | 2-VP (4-VP) (1h)* |
|---|---|---|---|---|---|---|
| Albumin | 66472 | 5.7 | 35 | 68% | 80% | 100% |
| Phosphorylase | 97158 | 6.8 | 36 | 65% | 78% | 100% |

| | | | | | | |
|---|---|---|---|---|---|---|
| ***The % of alkylation in the tree cases has been measured via mass spectrometry in the MALDI-TOF mode of the intact proteins.** | | | | | | |

Here too one can notice how, in the case of IAA, for both proteins, values of *ca.* 68% alkylation are reached after one hour of reaction, and barely of *ca*. 80% after six hours; conversely, in the case of 4VP (and its analogue 2VP) 100% reaction values are reached after only one hour of incubation.

### Example No. 6

*Alkylation of proteins with normal or deuterated 2VP or 4VP for quantitative analysis of protein expression.* For the study of variation of protein expression in control cells *vs.* cells treated with a variety of chemicals (e.g., drugs, inhibitors, promoters, etc.) it is important to be able to label the two cell populations, separately, with a normal ("light") or with a deuterated ("heavy") agent. The two samples are then mixed, in a 1:1 ratio, and the sample subjected to 2-D map analysis. The protein zones which, via analysis with quantitation programs (such as the PDQuest or Melanie), appear to have varied their expression (by showing increments or decrements of stain intensity, possibly reflecting up- or down-regulation in protein synthesis) are then eluted from the polyacrylamide gel and digested with trypsin. The resulting peptides are then analysed by mass spectrometry in the MALDI-TOF mode. At this point, all peptides marked with 2VP or 4VP are split into two peaks, the light and heavy ones (this last one marked with the deuterated isotope), spaced apart by 7 Da (in the case of hepta-deuterated pyridines) or by 4 Da (in the case in which only the pyridine ring, and not the vinyl moiety, is deuterated) or by 5 Da (in the case of partial deuteration of the vinyl moiety). A unit ratio between the areas of the two mono-isotopic peaks means that there has been no variation in protein synthesis between the control and treated cells. If, on the contrary, this ratio is higher (or lower) than one, this means that, in the treated cells, there has been an induction, or repression, of protein synthesis. This method thus permits to assess, in a precise and unambiguous manner, which effectors induce or repress protein synthesis and is thus fundamental in evaluating all biological effects induced by drugs, or the appearance of genetically induced diseases, onset of cancer etc. The use of isotopic ratios for these quantitative biological studies has already been described in the literature (Gygi, S.P., Aebersold, R., in *Proteomics: a Trend Guide,* Blackstock, W., Mann, M., eds., Elsevier, London, 2000, pp. 31-36), but the alkylating agent is a very complex molecule, the proper reactivity of which has not been demonstrated, and which is unable of alkylating -SH groups in a quantitative fashion, as in the case of 2VP and 4VP. In addition, the above agent (called ICAT, isotope coded affinity tag) is biotinylated, since this affinity label is needed for capturing by affinity chromatography only the marked peptides, out of an extremely heterogeneous peptide population. This is not necessary in the standard 2-D map analysis, since only intact proteins are separated, and not their peptide digest. We have thus prepared a 2-vinylpyridine either partially or totally deuterated, by using as starting material 2-methylpyridine hepta-deuterated, from which one can prepare the corresponding picolyl-lithium and then, via reaction with paraformaldehyde, obtain the 2-(2-hydroxyethyl)-pyridine, according to J. *Finkelstein et al.,* Journal of Organic Chemistry 4 (1939) 374-380. From this last compound one can obtain (I.C. Ivanov *et al*., Arch. Pharm. 322, 1989, 181-190) hepta-deuterated 2-vinylpyridine, when using d₂-paraformaldehyde, or penta-deuterated, when utilizing non-deuterated paraformaldehyde, thus with mass differences of 7 and 5 Da as compared with "light" (non-deuterated) 2-vinylpyridine. The synthesis of the other two (3VP and 4VP) deuterated vinylpyridines can be obtained via suitable deuterated precursors according to the syntheses described, e.g., in US patent 2.556.845, in the case of 4-vinylpyridine, or in Tetrahedron Letters 1993, 8329 and in Journal of the American Chemical Society 75, 1953, 4738, in he case of 3-vinylpyridine.

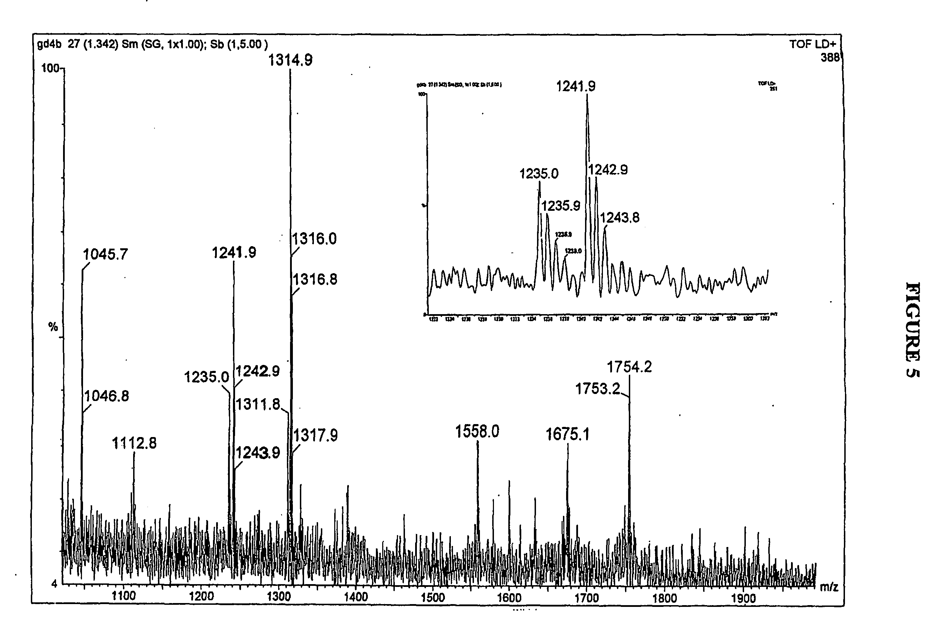

Figure 5 gives an example of this type of analysis. Sera from normal rats were separately marked with "light" and "heavy" (hepta-deuterated) 2-vinylpyridine, mixed in a 70% deuterated/30% light label, and separated by 2-D mapping. The zone of the α₁-acid glycoprotein (Mᵣ 21546, pI 5.0) was eluted, digested with trypsin and analysed by mass spectrometry in the MALDI-TOF mode. This analysis gave the mass spectrum of Fig. 5. A number of peptide fragments is observed at various m/z values, including the two fragments at m/z 1235.0 and 1241.9, The difference in m/z of 6.9 coincides with the difference between the 2-d₇-VP and the non-deuterated 2-VP. This observation, together with the ratio of the relative intensities of the two signals (ca. 68:32) confirms that this peptide contains a single Cys residue which reflects the ratio of the two isotopic markers (70:30) in the original protein mixture

## Claims

1. A process for use in the pre-treatment of a proteome derived protein mixture for use in a separation technique comprising electrophoretic two-dimensional maps, said maps comprising a first dimension by isoelectric focusing (either conventional or in immobilized pH gradients) followed by a second SDS-PAGE dimension, the process comprising the selective alkylation of -SH groups in a protein, by the reaction in neutral or alkaline milieu of said protein in a mixture comprising at least one chaotropic agent and at least one zwitterionic, neutral or anionic surfactant with a vinylpyridine
wherein the percent alkylation of the total -SH groups is higher than 90 %.

2. A process according to claim 1, wherein the vinylpyridine is selected from 2-vinylpyridine, 3-vinylpyridine or 4-vinylpyridine.

3. A process according to claim 2, wherein the vinylpyridine is selected from 2-vinylpyridine and 4-vinylpyridine.

4. A process according to claim 2 or 3 wherein the vinylpyridine are deuterated, either partially or totally.

5. A process according to any one of claims from 1 to 4, wherein the percent alkylation of the total -SH groups is higher than 95 %.

6. A process according to claim 5, wherein the percent alkylation of the total -SH groups is about 100 %.

## Patentansprüche

1. Verfahren zur Verwendung bei der Vorbehandlung einer von Proteom derivierten Proteinmischung zur Verwendung bei einer Trennungstechnik, umfassend elektrophoretische zweidimensionale Karten, wobei die Karten eine erste Dimension durch isoelektrisches Fokussieren (entweder auf herkömmliche Weise oder in immobilisierten pH-Gradienten) gefolgt von einer zweiten SDS-PAGE-Dimension umfassen, wobei das Verfahren die selektive Alkylierung von -SH-Gruppen in einem Protein durch Reaktion des Proteins in neutralem oder alkalischem Medium in einer Mischung umfassend mindestens ein chaotropes Mittel und mindestens ein zwitterionisches, neutrales oder anionisches Tensid mit einem Vinylpyiridin umfasst,
wobei der Prozentsatz der Alkylierung der gesamten - SH-Gruppen höher als 90 % liegt.

2. Verfahren nach Anspruch 1, wobei das Vinylpyridin unter 2-Vinylpyridin, 3-Vinylpyridin oder 4-Vinylpyridin ausgewählt wird.

3. Verfahren nach Anspruch 2, wobei das Vinylpyridin unter 2-Vinylpyridin und 4-Vinylpyridin ausgewählt wird.

4. Verfahren nach Anspruch 2 oder 3, wobei das Vinylpyridin entweder teilweise oder vollständig deuteriert ist.

5. Verfahren nach einem der Ansprüche 1 bis 4, wobei der Prozentsatz der Alkylierung der gesamten -SH-Gruppen höher als 95 % liegt.

6. Verfahren nach Anspruch 5, wobei der Prozentsatz der Alkylierung der gesamten -SH-Gruppen etwa 100 % beträgt.

## Revendications

1. Procédé destiné à une utilisation dans le prétraitement d'un mélange protéique dérivé d'un protéome pour une utilisation dans une technique de séparation comprenant des cartes électrophorétiques bidimensionnelles, lesdits cartes comprenant une première dimension par focalisation iso-électrique (soit conventionnelle, soit sur gradients de pH immobilisés) suivie d'une seconde dimension par la SDS-PAGE, le procédé comprenant l'alkylation sélective de groupes -SH dans une protéine, par la réaction en milieu neutre ou alcalin de ladite protéine dans un mélange comprenant au moins un agent chaotropique et au moins un agent tensioactif zwitterionique, neutre ou anionique avec une vinylpyridine,
dans lequel l'alkylation des groupes -SH totaux exprimée en pourcentage est supérieure à 90 %.

2. Procédé selon la revendication 1, dans lequel la vinylpyridine est choisie parmi la 2-vinylpyridine, la 3-vinylpyridine et la 4-vinylpyridine.

3. Procédé selon la revendication 2, dans lequel la vinylpyridine est choisie parmi la 2-vinylpyridine et la 4-vinylpyridine.

4. Procédé selon la revendication 2 ou 3, dans lequel la vinylpyridine est deutériée, soit partiellement, soit totalement.

5. Procédé selon l'une quelconque des revendications 1 à 4, dans lequel l'alkylation des groupes -SH totaux exprimée en pourcentage est supérieure à 95 %.

6. Procédé selon la revendication 5, dans lequel l'alkylation des groupes -SH totaux exprimée en pourcentage est d'environ 100 %.
